Europäisches Patentamt

European Patent Office

Office européen des brevets

(11) Publication number: **0 027 004**
**B1**

(12) **EUROPEAN PATENT SPECIFICATION**

(45) Date of publication of patent specification: **15.08.84**

(51) Int. Cl.³: **C 07 C 85/06, C 07 C 87/54**

(21) Application number: **80303344.8**

(22) Date of filing: **24.09.80**

(54) Method for producing unsymmetrical diphenylamines.

(30) Priority: **03.10.79 US 81308**

(43) Date of publication of application:
**15.04.81 Bulletin 81/15**

(45) Publication of the grant of the patent:
**15.08.84 Bulletin 84/33**

(84) Designated Contracting States:
**DE GB IT NL**

(56) References cited:
**DE - C - 241 853**
**GB - A - 809 752**
**US - A - 1 885 355**

(73) Proprietor: **XEROX CORPORATION**
**Xerox Square - 020**
**Rochester New York 14644 (US)**

(72) Inventor: **Szabo, Paul**
**39 Richview road Apt. 1811**
**Islington Ontario M9A 4M7 (CA)**
Inventor: **Martin, Trevor I.**
**502 Shannon Crescent**
**Burlington Ontario L7L 2R8 (CA)**
Inventor: **Freeman, Daniel E.**
**1440 Bloor Street East Apt. 209**
**Mississauga Ontario L4X 1R5 (CA)**
Inventor: **Lennon, John M.**
**2150 Roche Court Apt. 807**
**Mississauga Ontario L5K 1T5 (CA)**

(74) Representative: **Cline, Roger Ledlie et al,**
**STANLEY, POPPLEWELL, POOLE 57 Lincoln's Inn**
**Fields**
**London WC2A 3LS (GB)**

Courier Press, Leamington Spa, England.

## Description

This invention relates to the preparation of unsymmetrical, substituted diphenylamines, especially 3-methyldiphenylamine.

There has long been known procedures for the production of symmetrical, unsubstituted diphenylamines. Aniline is normally condensed in the vapor phase in the presence of a catalyst. Such processes are disclosed in U.S. Patents 3,079,439 and 3,118,944. The production of diphenylamine from aniline in the liquid phase is disclosed in U.S. Patent 3,205,265 wherein the reaction is catalyzed by a halogen and an oxygen containing compound of phosphorous.

It has also been disclosed in U.S. Patent Specification No. 1,885,355 that a symmetrical unsubstituted diamine such as N,N' di-beta-naphthyl-p-phenylenediamine could be prepared by reacting a diamine such as p-phenylenediamine with an excess of a substituted fused benzene ring reactant such as beta-napthol.

More recently, there has been disclosed a process for the manufacture of diarylamines by the reaction of a phenol and aniline in the presence of a small concentration of sulfonic acid. Such process is disclosed in German Patent 2,042,774. This patent suggests the manufacture of both symmetrical and unsymmetrical substituted diphenylamines in the presence of the acid catalyst. A continuous process is suggested through the use of interconnected autoclaves in succession whereby each autoclave is outfitted with a distillation column for the removal of water produced in the reaction. According to this process, the reaction operates with an excess of aniline. Under these conditions, the self-condensation of aniline leads to the formation of a substantial amount of by-product which is diphenylan ine.

In yet another process described in Canadian Patent No. 1,005,462, there is described a process for the production of diarylamines by the reaction of naphthol and a primary arylamine. In such process, ferric chloride is utilized as a catalyst. As in similar prior art, the primary arylamine is utilized in excess and therefore the formation of by-product by self-condensation of the primary arylamine is significant.

German Patent Specification No. 241,853 is directed towards the use of catalysts in producing diarylamines and discloses, inter alia, the reaction of napthol with chloroaniline.

In all of the prior art, there is great tolerance for impurities in the final product, usually diphenylamine resulting from the self-condensation of aniline. The presence of such an impurity was probably of little concern because the end use of the product did not require high purity. However, there is a need to provide unsymmetrical diarylamines in high yield and purity when such amines are utilized as precursors for other products.

In accordance with the present invention, there is provided a process for preparing an alkyl substituted unsymmetrical diphenylamine by the reaction of a primary phenylamine and a phenol in the presence of a catalyst, characterized in that one of reactant species is alkyl substituted and the other is unsubstituted, the phenol is maintained in excess of the primary phenylamine and the reaction is carried out at an elevated temperature and pressure.

Subsequent to completion of the reaction, the unreacted phenolic compound may be recovered and recycled for reaction with additional primary phenylamine. Surprisingly, the process provides for the suppression of the self-condensation of the primary phenylamine while still providing for a high yield of the desired unsymmetrical substituted diphenylamine.

The process of this invention successfully achieves suppression of the traditional problem of self-condensation of primary phenylamines by employing an excess of the phenolic compound in the reaction mixture. Generally, the molar ratio of primary phenylamine to the phenolic compound is in the range of from .1:1 to .7:1. However, a molar ratio of primary phenylamine to phenolic compound of about .3:1 has been found to be optimum. The excess phenolic compound is subsequently recovered easily from the reaction mixture for reuse in subsequent production of the desired substituted, unsymmetrical diphenylamine. By the recycling step, a high yield and overall conversion is achieved even though low conversion is experienced in each individual reaction. A catalyst has been found to be useful in the process of this invention to increase the rate of reaction between the phenolic compound and the primary amine. Any suitable catalyst can be employed, preferably ferric chloride. Other catalysts include benzene-sulfonic acid, toluene sulfonic acid, sulfuric acid, and acid alumina. The amount of catalyst utilized is usually in the range of from 1 to 15 percent by weight of both reactants, although about 5 percent by weight has been found to be most efficient.

Various alkyl substituted or unsubstituted primary phenylamines can be employed in the process of this invention. For the particular case of 3-methyldiphenylamine, aniline is preferred because of its availability and low cost. Other suitable primary phenylamines include alkyl substituted anilines, such as the toluidines and xylidines.

The phenolic compounds of most interest include alkyl substituted or unsubstituted phenolic compounds, a preferred group of phenols being the cresols. For the particular case of 3-methyldiphenylamine, m-cresol is preferred.

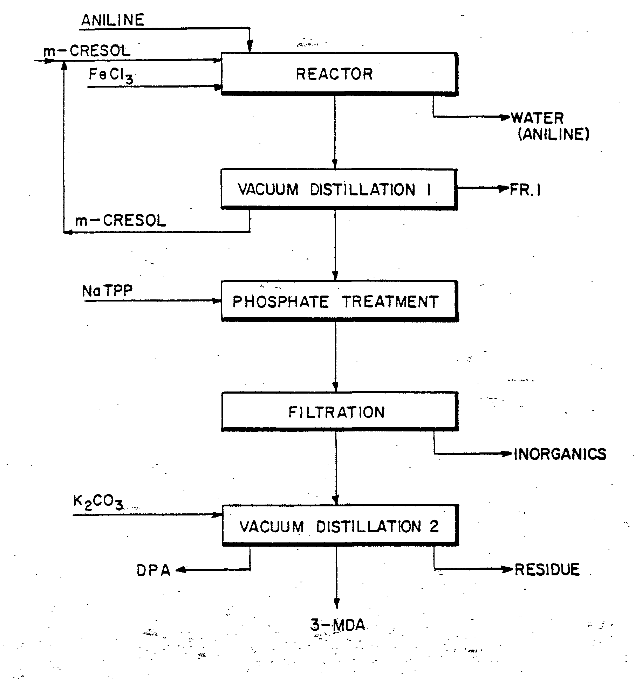

Figure 1 provides a flow chart outlining the various steps in the process of this invention.

A typical reaction involves aniline and m-cresol with ferric chloride as the catalyst. Accordingly, in Figure 1, a pressure reactor system equipped with a stirrer is charged with about 5 moles of aniline, 16.6 moles of m-cresol and about .71 moles of anhydrous ferric chloride. The reaction is typically operated under an inert atmosphere of nitrogen at a temperature in the range of 280°C to 350°C. Since water is produced during the reaction, a control valve is utilized to periodically vent the system allowing water to be removed during the reaction as it accumulates. The reaction proceeds for 1 to 6 hours depending on the operating temperature. As the reaction proceeds, the pressure oscillates in the narrow range of the control valve setting as water is removed. A small amount of aniline escapes with the water but is inconsequential and can be easily recovered. At the end of the reaction, the reactor is cooled and the entire contents of the reactor are transferred to a vacuum distillation system. The catalyst could be removed by filtration prior to distillation. The first fraction, as indicated in Figure 1 by Fr. 1, is removed and contains water with traces of m-cresol and aniline. This is discarded and a second fraction is obtained containing about 98 percent m-cresol with traces of aniline, diphenylamine and 3-methyldiphenylamine. This fraction is reused as the m-cresol for the next batch as indicated in Figure 1.

The residue from the vacuum distillation is then treated with a phosphate to remove traces of iron from the product. In this illustrative procedure, about .5 parts by weight of sodium tripolyphosphate is added to 1 part residue. The mixture is held at about 100°C for about 1 hour with stirring. This mixture is then filtered to remove the inorganic salts as indicated in Figure 1 with the filtrate being transferred to a vacuum distillation system. The phosphate treatment is required only if traces of iron are detrimental to the quality of the final product. It has been found that a small amount of potassium carbonate added to this second distillation system results in the production of a colorless product. An intermediate fraction is obtained containing traces of m-cresol, aniline, diphenylamine and a slight amount of 3-methyldiphenylamine. This is indicated in Figure 1 as "DPA" and may be combined with subsequent batches for recovery of the useful material therein. The main fraction is recovered at a temperature in the range of from 170°C to 180°C under 0.013 mbar ($10^{-2}$ mm of mercury vacuum) to provide 3-methyldiphenylamine in excess of 97 percent purity. A small amount of diphenylamine is also present. The diphenylamine content of the final product is dependent upon the efficiency of the distillation unit. The residue is easily removed from the distillation system with water.

As indicated above, the excess phenolic compound is recycled thereby providing a high yield and overall conversion while at the same time providing a highly pure product.

## Claims

1. A process for preparing an alkyl substituted unsymmetrical diphenylamine by the reaction of a primary phenylamine and a phenol in the presence of a catalyst, characterized in that one of reactant specis is alkyl substituted and the other is unsubstituted, the phenol is maintained in excess of the primary phenylamine and the reaction is carried out at an elevated temperature and pressure.

2. A process as claimed in claim 1, wherein the molar ratio of primary phenylamine to phenol is in the range from .1:1 to .7:1.

3. A process as claimed in claim 2, wherein the molar ratio of the primary phenylamine to phenol is approximately .3:1.

4. A process as claimed in any one of claims 1 to 3, wherein the excess phenol is recovered from the reaction product and recycled to said process.

5. A process as claimed in any one of claims 1 to 4, wherein the catalyst is ferric chloride.

6. A process as claimed in any one of claims 1 to 5, wherein the primary phenylamine is aniline.

7. A process as claimed in any one of claims 1 to 5, wherein the phenol is an alkyl substituted phenol.

8. A process as claimed in claim 7, wherein the phenol is a cresol.

9. A process as claimed in claim 8, wherein the cresol is m-cresol.

## Revendications

1. Procédé pour préparer une diphénylamine non symétrique à substitution alkyle par la réaction d'une phénylamine primaire et d'un phénol en présence d'un catalyseur, caractérisé en ce qu'une des espèces de produits réagissants est à substitution alkyle et l'autre est non substituée, le phénol est maintenu en excés par rapport à la phénylamine primaire et la réaction est réalisée à une température et sous une pression élevées.

2. Procédé selon la revendication 1, dans lequel le rapport molaire entre la phénylamine primaire et le phénol est dans la gamme de 0,1:1 à 0,7:1.

3. Procédé selon la revendication 2, dans lequel le rapport molaire entre la phénylamine primaire et le phénol est approximativement 0,3:1.

4. Procédé selon l'une quelconque des revendications 1 à 3, dans lequel l'excès de phénol est récupéré à partir du produit réactionnel et recyclé dan le procédé.

5. Procédé selon l'une quelconque des revendications 1 à 4, dans lequel le catalyseur est du chlorure ferrique.

6. Procédé selon l'une quelconque des revendications 1 à 5, dans lequel la phénylamine primaire est l'aniline.

7. Procédé selon l'une quelconque des revendications 1 à 5, dans lequel le phénol est un phénol à substitution alkyle.

8. Procédé selon la revendication 7, dans lequel le phénol est un crésol.

9. Procédé selon la revendication 8, dans lequel le crésol est du m-crésol.

**Patentansprüche**

1. Verfahren zur Herstellung eines alkylsubstituierten, unsymmetrischen Diphenylamins durch Umsetzung eines primären Phenylamins und eines Phenols in Gegenwart eines Katalysators, dadurch gekennzeichnet, daß einer der Reaktanten alkylsubstituiert und der andere unsubstituiert ist, das Phenol bezüglich dem primären Phenylamin im Überschuß gehalten und die Umsetzung bei erhöhter Temperatur und Druck durchgeführt wird.

2. Verfahren nach Anspruch 1, dadurch gekennzeichnet, daß das Molverhältnis von primärem Phenylamin zu Phenol im Bereich 0,1:1 bis 0,7:1 liegt.

3. Verfahren nach Anspruch 2, dadurch gekennzeichnet, daß das Molverhältnis von primärem Phenylamin zu Phenol etwa 0,3:1 beträgt.

4. Verfahren nach einem der Ansprüche 1 bis 3, dadurch gekennzeichnet, daß das überschüssige Phenol aus dem Reaktonsprodukt rückgewonnen und dem Verfahren rückgeführt wird.

5. Verfahren nach einem der Ansprüche 1 bis 4, dadurch gekennzeichnet, daß der Katalysator Eisen (III) — chlorid ist.

6. Verfahren nach einem der Ansprüche 1 bis 5, dadurch gekennzeichnet, daß das primäre Phenylamin Anilin ist.

7. Verfahren nach einem der Ansprüche 1 bis 5, dadurch gekennzeichnet, daß das Phenol ein alkylsubstitutiertes Phenol ist.

8. Verfahren nach Anspruch 7, dadurch gekennzeichnet, daß das Phenol ein Cresol ist.

9. Verfahren nach Anspruch 8, dadurch gekennzeichnet, daß das Cresol m-Cresol ist.

ANILINE

m−CRESOL

FeCl$_3$

REACTOR

→ WATER (ANILINE)

VACUUM DISTILLATION 1 → FR.I

m−CRESOL

NaTPP → PHOSPHATE TREATMENT

FILTRATION

→ INORGANICS

K$_2$CO$_3$ → VACUUM DISTILLATION 2

DPA ←

→ RESIDUE

3−MDA

1